# EUROPEAN PATENT SPECIFICATION

(11) **EP 2 214 483 B1**
(45) Date of publication and mention of the grant of the patent: **20.06.2012**
(21) Application number: 08841518.7
(22) Date of filing: 24.10.2008
(51) Int. Cl.: G01N 33/68, C12Q 1/34

(54) **METHODS OF IDENTIFYING HISTONE DEACETYLASE INHIBITORS USEFUL FOR NEUROLOGICAL DISORDERS**
VERFAHREN ZUM IDENTIFIZIEREN VON HISTONDEACETYLASE-HEMMERN, DIE SICH FÜR NEUROLOGISCHE ERKRANKUNGEN EIGNEN
PROCÉDÉS POUR IDENTIFIER DES INHIBITEURS D'HISTONE DÉSACÉTYLASE UTILES POUR DES TROUBLES NEUROLOGIQUES

(30) Priority: 26.10.2007 US 982882 P
(43) Date of publication of application: 11.08.2010
(73) Proprietor: REPLIGEN CORPORATION, Waltham, MA 02453 (US)
(72) Inventor: RUSCHE, James R., Framingham Massachusetts 01702 (US); COOPER, Andrew, Lawrence Massachusetts 01843 (US)
(74) Representative: Peterreins, Frank
(86) International application number: PCT/US2008/081121
(87) International publication number: WO 2009/055675

(56) References cited:
- WO-A1-2007/058927
- US-A1- 2004 072 849
- US-A1- 2007 078 083
- US-A1- 2007 123 452
- US-A1- 2007 219 244
- GOTTESFELD ET AL: "Small molecules affecting transcription in Friedreich ataxia", PHARMACOLOGY AND THERAPEUTICS, ELSEVIER, GB, vol. 116, no. 2, 26 September 2007 (2007-09-26), pages 236-248, XP022272070, ISSN: 0163-7258, DOI: DOI:10.1016/J.PHARMTHERA.2007.06.014
- HERMAN ET AL.: 'Histone Deacetylase Inhibitors Reverse Gene Silencing In Friedreich's Ataxia.' NATURE CHEMICAL BIOLOGY vol. 2, no. 10, October 2006, pages 551 - 558, XP002424074

## Description

### TECHNICAL FIELD

This invention relates to methods of identifying compounds useful for treatment of neurological conditions.

### BACKGROUND

Friedreich's ataxia (FRDA) is the most prevalent inherited ataxia in Caucasians (see Pandolfo (1999) Semin. Neurol., 19:311). Individuals with FRDA have a deficiency of the mRNA encoding frataxin, a highly conserved 210 amino acid nuclear-encoded, mitochondrial protein that is thought to be involved in iron homeostasis, storage, and transfer of iron-sulfur clusters to partner proteins such as aconitase (see Bulteau et al. (2004) Science, 305:242; Seznec et al. (2005) Hum. Mol. Genet., 14:463; Calabrese et al. (2005) J. Neurol. Sci., 233:145).

Frataxin insufficiency leads to progressive spinocerebellar neurodegeneration resulting in gait and hand in-coordination, slurred speech, muscle weakness and sensory loss with extraneural scoliosis, cardiomyopathy, and diabetes. Generally within 15 to 20 years after the first appearance of symptoms, affected individuals are confined to a wheelchair and in later stages, become completely incapacitated. Most affected individuals die in early adulthood of heart disease. Although Antioxidant- and iron-chelator-based strategies have been used to treat FRDA, these strategies treat only the symptoms of the disease and not the cause, i.e., frataxin deficiency. Therefore, there is a need to develop molecules that could restore frataxin protein expression for the treatment of a neurological condition such as FRDA.

The DNA abnormality found in 98% of FRDA patients is an unstable hyper-expansion of a GAA triplet repeat in the first intron of the frataxin gene (see Campuzano et al., Science 271:1423 (1996)). Triplet repeat expansion in genomic DNA is associated with many other neurological conditions (e.g., neurodegenerative and neuromuscular diseases) including myotonic dystrophy, spinal muscular atrophy, fragile X syndrome, Huntington's disease, spinocerebellar ataxias, amyotrophic lateral sclerosis, Kennedy's disease, spinal and bulbar muscular atrophy, and Alzheimer's disease. Triplet repeat expansion may cause disease by altering gene expression. For example, in Huntington's disease, spinocerebellar ataxias, fragile X syndrome, and myotonic dystrophy, expanded repeats lead to gene silencing. Therefore, there is a need to identify and develop molecules that could restore the normal function of genes in neurological conditions.

WO 2007/058927 discloses HDAC inhibitors for i.a. the treatment of Friedreich's ataxia.

US 2007/0219244 discloses HDAC inhibitors that may be used as therapeutics for the treatment of a neurodegenerative or neuromuscular condition inhibitors.

Gottesfeld, Pharmacology & Therapeutics, 116 (2007) 236-248 reviews the development of small molecule therapeutics for treating Friedreich ataxia.

US 2007/123452 discloses a method of selecting an IL-2 production inhibitor and/or an immunocyte proliferation inhibitor having low GATA-1 production inhibitory activity, which comprises measuring the HDAC4 and/or HDAC8 inhibitory activity of a test substance, and a method of selecting an immunosuppressant having low thrombocytopenic activity, which comprises measuring the HDAC4 and/or HDAC8 inhibitory activity of a test HDAC inhibitor.

Herman et al, Nature Chemical Biology, 2(10), 2006, 551-558, disclose HDAC inhibitors for reverse gene silencing in Friedreich's ataxia.

US 2004/072849 discloses compounds capable of inhibiting histone deacetylatase activity.

### SUMMARY

The invention in its broadest sense is defined by the appended claims.

This invention is based, inter alia, on the surprising discovery that compounds that are specific inhibitors of histone deacetylase 3 (HDAC3) also increase frataxin expression. Accordingly, the invention provides methods of identifying specific inhibitors of HDAC3 that can be useful for treatment of neurological conditions associated with inhibition of gene expression by HDAC3 activity, e.g., Friedreich's ataxia.

Specific inhibitors of HDAC3 provide advantages for treatment of neurological conditions over the use of broad-spectrum HDAC inhibitors by reducing toxicities associated with inhibition of other HDACs and focusing inhibition on an HDAC capable of increasing expression of genes reduced in expression in the diseased cells. Such specific HDAC3 inhibitors provide a higher therapeutic index, resulting in better tolerance by patients during chronic or long-term treatment.

In one aspect, the invention features methods of identifying a candidate compound for treatment of a neurological condition that by obtaining a test compound; assaying a first activity of the test compound to inhibit histone deacetylase activity of a histone deacetylase 3 (HDAC3); assaying a second activity of the test compound to inhibit histone deacetylase activity of a class I histone deacetylase other than the HDAC3 (e.g., HDAC1, HDAC2, or HDAC8); and identifying the test compound as a candidate compound for treatment of a neurological condition associated with a frataxin deficiency if the first activity of the test compound is greater than the second activity of the test compound.

In another aspect, the invention features methods of identifying a candidate compound for treatment of a neurological condition by obtaining a test compound; assaying a first activity of the test compound to inhibit histone deacetylase activity of a HDAC3; assaying a second activity of the test compound to inhibit histone deacetylase activity of a HDAC1; assaying a third activity of the test compound to inhibit histone deacetylase activity of a HDAC2; assaying a fourth activity of the test compound to inhibit histone deacetylase activity of a HDAC8; and identifying the test compound as a candidate compound for treatment of a neurological condition if the first activity of the test compound is greater than each of the second, third, and fourth activities of the test compound.

In a further aspect, the invention features methods of identifying a candidate compound for treatment of a neurological condition by obtaining a test compound; assaying a first activity of the test compound to inhibit histone deacetylase activity of a HDAC3; assaying a second activity of the test compound to inhibit histone deacetylase activity of a class I or class II histone deacetylase other than the HDAC3 (e.g., HDAC1, HDAC2, HDAC4, HDAC5, HDAC6, HDAC7, HDAC8, HDAC9, or HDAC10); and identifying the test compound as a candidate compound for treatment of a neurological condition associated with a frataxin deficiency if the first activity of the test compound is greater than the second activity of the test compound.

In another aspect, the invention features methods of identifying a candidate compound for treatment of a neurological condition by obtaining a test compound; assaying an activity of the test compound to inhibit histone deacetylase activity of a HDAC3; assaying a set of activities of the test compound to inhibit histone deacetylase activity of each of histone deacetylases 1, 2, 4, 5, 6, 7, 8, 9, and 10; and identifying the test compound as a candidate compound for treatment of a neurological condition if the first activity of the test compound is greater than each activity of the set of activities of the test compound.

In some embodiments of the above methods, one or more of the HDACs (e.g., HDAC3) is a human HDAC (e.g., a human HDAC3).

In some embodiments of the above methods, the test compound is identified as a candidate compound for treatment of a neurological condition if the first activity is at least about 1.5-fold greater (e.g., at least about 2-fold, 3-fold, 4-fold, 5-fold, 10-fold, 15-fold, or 20-fold greater) than another activity (e.g., the second, third, or fourth activity, or each activity of the set of activities).

In some embodiments of the above methods, the neurological condition is Friedreich's ataxia, myotonic dystrophy, spinal muscular atrophy, fragile X syndrome, Huntington's disease, a spinocerebellar ataxia, Kennedy's disease, amyotrophic lateral sclerosis, spinal and bulbar muscular atrophy, or Alzheimer's disease. In some embodiments of the above methods, the neurological condition is associated with expansion of a triplet repeat (e.g., Friedreich's ataxia, myotonic dystrophy, spinal muscular atrophy, fragile X syndrome, Huntington's disease, a spinocerebellar ataxia, or Kennedy's disease).

In some embodiments, the above methods further include assaying the activity of the candidate compound to increase expression of one or more genes whose expression is decreased in the neurological condition (e.g., frataxin, huntingtin, brain derived neurotrophic factor *(BDNF),* peroxisome proliferator-activated receptor-gamma, coactivator 1, alpha *(PGC1A),* ataxin, fragile X mental retardation *(FMR1),* dystrophia myotonica protein kinase *(DMPK),* or androgen receptor).

In some embodiments, the above methods further include assaying the general cellular toxicity of the candidate compound, e.g., by assaying the toxicity of the candidate compound on a cell line (e.g., HepG2).

In some embodiments, the above methods are repeated for a plurality of test compounds (e.g., at least 10, 20, 50, 100, 200, 500, or 1000 test compounds).

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

In addition, the materials, methods, and examples are illustrative only and not intended to be limiting.

Other features and advantages of the invention will be apparent from the following detailed description, and from the claims.

### DESCRIPTION OF THE DRAWING

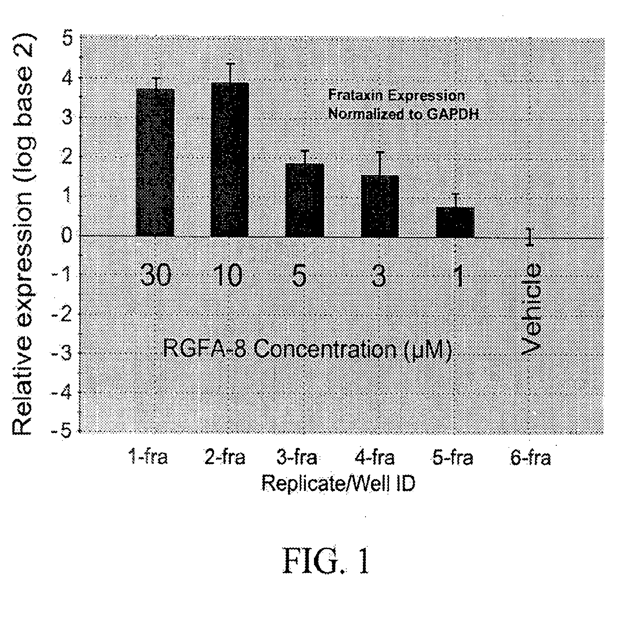

FIG. 1 is a bar graph depicting fold-upregulation of frataxin mRNA expression in human cells after administration of the indicated concentrations of the HDAC3-specific histone deacetylase inhibitor RGFA8.

### DETAILED DESCRIPTION

The invention relates to the surprising discovery that specific histone deacetylase 3 (HDAC3) inhibitors also increase expression of frataxin, and could therefore be useful in the treatment of neurological conditions (e.g., neurological conditions associated with reduced frataxin expression). Accordingly, the invention provides methods of identifying compounds that could be used as therapeutics for various chronic and acute neurological conditions such as, for example, Friedreich's ataxia.

### Histone Deacetylase Inhibitors

The DNA abnormality found in 98% of FRDA patients is an unstable hyper-expansion of a GAA triplet repeat in the first intron of the frataxin gene that results in a defect in transcription of the frataxin gene (see Campuzano et al., 1996, Science, 271:1423-27). FRDA patients have a marked deficiency of frataxin mRNA, and the longer the GAA triplet repeats, the more profound the frataxin deficiency. FRDA is typical of triplet repeat diseases: normal alleles have 6-34 repeats while FRDA patient alleles have 66-1700 repeats. Longer GAA triplet repeats are associated with earlier onset and increased severity of the disease. The invention provides methods of identifying specific HDAC3 inhibitors that can restore gene function in a neurological disease that is associated with expansion of a triplet repeat, such as FRDA or Huntington's disease. For example, HDAC3 inhibitors identified by the methods described herein increase frataxin mRNA and protein in lymphocytes from FRDA patients. A "histone deacetylase 3 (HDAC3) inhibitor" is a small molecule that binds to HDAC3 to modulate the levels of acetylation of histones, non-histone chromosomal proteins, and other cellular proteins. A HDAC3 inhibitor identified by the methods described herein may interact with a HDAC3 to modulate the level of acetylation of cellular targets.

A HDAC, as described herein, can be any polypeptide having features characteristic of polypeptides that catalyze the removal of the acetyl group (deacetylation) from acetylated target proteins. Features characteristic of HDACs are known in the art, see, for example, Finnin et al., 1999, Nature, 401:188. Thus, a HDAC can be a polypeptide that represses gene transcription by deacetylating the *ε*-amino groups of conserved lysine residues located at the N-termini ofhistones, e.g., H3, H4, H2A, and H2B, that form the nucleosome. HDACs also deacetylate other proteins such as p53, E2F, α-tubulin, and MyoD. See Annemieke et al., 2003, Biochem. J., 370:737. HDACs can also be localized to the nucleus, and certain HDACs can be found in both the nucleus and also the cytoplasm.

HDAC3 inhibitors identified by the methods described herein may interact with any HDAC. However, the HDAC3 inhibitors will have at least about 2-fold (e.g., at least about 5-fold, 10-fold, 15-fold, or 20-fold) greater activity to inhibit HDAC3 as compared to one or more other HDACs (e.g., one or more HDACs of class I or class II). Class I HDACs are those that most closely resemble the yeast transcriptional regulator RPD3. Examples of class I HDACs include HDACs 1, 2, 3 and 8, as well as any HDAC that has a deacetylase domain exhibiting from 45 % to 93 % identity in amino acid sequence to HDACs 1, 2, 3 and 8. Class II HDACs are those that most closely resemble the yeast HDAC1 enzyme. Examples of class II HDACs include HDACs 4, 5, 6, 7, 9 and 10.

### Assaying Test Compounds

In certain aspects, HDAC3 inhibitors are found by identifying test compounds (e.g., from a group of test compounds) that inhibit the activity of HDAC3 more, e.g., 2, 3, 4, 5, 10, or more times, than they inhibit the activity one or more other HDACs. HDAC inhibitory activity of test compounds can be assayed by standard means. Briefly, an assay typically involves incubating an acetylated HDAC substrate with a HDAC enzyme in the presence or absence of a test compound and detecting the removal of acetyl groups from the substrate. HDAC inhibition assays can be performed, e.g., in a cell, in a cell extract, or in a cell-free mixture. Exemplary HDAC inhibition assays are described in Pérez-Balado et al., 2007, J. Med. Chem., 50:2497-2505; Herman et al., 2006, Nat. Chem. Biol., 2:551-558; Beckers et al., 2007, Int. J. Cancer, 121:1138-48, and Khan et al., 2007, Biochem J., 0:BJ20070779. HDAC assay kits are commercially available from BIOMOL (Plymouth Meeting, PA) and Upstate (Charlottesville, VA). A small molecule microarray method for screening for HDAC inhibitors is described in Vegas et al., 2007, Angew. Chem. Int. Ed. Engl., 0:anie.200703198.

HDAC3 and other HDAC enzymes can be provided, e.g., as purified proteins, partially purified proteins, purified recombinant proteins, in cells, or cell extracts. Purification or partial purification of HDAC3 and other HDAC enzymes can be performed by standard means, including affinity chromatography and immunoprecipitation.

The HDAC substrate can be a commercially available substrate (e.g., Fluor de Lys™, BIOMOL) or an acetylated cellular HDAC substrate, e.g., histone H2A, histone H2B, histone H3, histone H4, α-tubulin, NFκB-3, or p53. Exemplary substrates further include acetylated peptides of the preceding proteins, e.g., residues 2-24 or 1-18 of Histone H4.

The deacetylation of the HDAC substrate can be detected by standard means. Commercially available substrates are provided with fluorimetric or colorimetric reagents that detect deacetylated lysines. In other aspects, the substrate can be ³H-acetylated, and deacetylation is detected by measuring the release of ³H from the substrate. In further aspects, antibodies can be used to distinguish acetylated substrates from deacetylated substrates. For example, antibodies specific for acetylated α-tubulin are available from Sigma, and antibodies specific for acetylated histone H3 are available from Upstate.

Compounds identified as HDAC3 inhibitors can be further tested for induction of expression of one or more genes that are underexpressed in a neurological disorder, e.g., frataxin (GenBank Accession No. NM_000144.3), huntingtin (GenBank Accession No. NM_002111.6), brain derived neurotrophic factor *(BDNF;* GenBank Accession No. NM_170735.4), peroxisome proliferator-activated receptor-gamma, coactivator 1, alpha *(PGC1A;* GenBank Accession No. NM_013261.3), ataxins (e.g., ataxin 1 (GenBank Accession No. NM_000332.2), fragile X mental retardation *(FMR1;* GenBank Accession No. NM_002024.3), dystrophia myotonica protein kinase *(DMPK;* GenBank Accession No. NM_004409.3), or androgen receptor (GenBank Accession No. NM_000044.2). Listed GenBank accession numbers indicate exemplary human cDNA sequences and are not meant to be limiting. Sequences of other alleles or alternatively spliced versions can also be used.

Typically, the inhibitor is administered to a cell or cell-free extract that expresses a nucleic acid or protein product of the gene, and the expression of the gene product is compared to its expression in the absence of the inhibitor. Any cells can be used, including primary cells obtained from a subject (e.g., a subject having a neurological disorder) or cells of a cell line. Exemplary cells include neural cells, neuronal cells, and lymphocytes. The cells can be isolated and stored frozen in aliquots to provide ease in scaling the assay to allow multiple samples or multiple assays to be done with the same cell source. In one embodiment, the cells are lymphocytes (e.g., derived from Friedreich's ataxia patients), which are primary cells or cells of a lymphoblastoid cell line.

Determination of the expression of nucleic acid and protein gene products can be accomplished by any of several standard methods. Nucleic acid expression can be determined, e.g., by hybridization (e.g., northern blotting), nucleic acid microarrays, PCR (e.g., reverse transcription-PCR (RT-PCR) or quantitative RT-PCR), primer extension, serial analysis of gene expression, nuclease protection assays, or reporter gene constructs. Protein expression can be determined, e.g., by immunoblotting (e.g., western blotting), immunoprecipitation, immunosorbent assay (e.g., ELISA or RIA), peptide microarrays, or fusion proteins (e.g., GFP fusions).

Useful compounds for chronic use will inhibit HDAC3 at concentrations that do not show significant cytotoxic activity. Cytotoxic activity can be measured by incubating compounds with an indicator cell line (e.g., the human transformed liver cell HepG2). Viable cell number is determined after an incubation period, typically between 24-72 hours following administration of the compound. Viable cells can be determined by many methods including but not limited to cell counting or using a substrate converted to a colored product by live cells such as MTS. The ratio of HDAC3 activity to cytotoxicity can identify molecules that increase expression of gene products reduced by disease and are tolerable to administration over long periods of time.

### Test Compounds

The new methods can be used to identify compounds, e.g., small organic or inorganic molecules (e.g., with a molecular weight less than 1,000 Da), oligopeptides, oligonucleotides, or carbohydrates, that inhibit HDAC3, e.g., to a greater extent than one or more other HDACs. In certain embodiments, screens of the present invention utilize libraries of test compounds. As used herein, a "test compound" can be any chemical compound, for example, a macromolecule (e.g., a polypeptide, a protein complex, glycoprotein, or a nucleic acid) or a small molecule (e.g., an amino acid, a nucleotide, an organic or inorganic compound). A test compound can have a formula weight of less than about 10,000 grams per mole, less than 5,000 grams per mole, less than 1,000 grams per mole, or less than about 500 grams per mole. The test compound can be naturally occurring (e.g., an herb or a natural product), synthetic, or can include both natural and synthetic components. Examples of test compounds include antioxidants, compounds that structurally resemble antioxidants, peptides, peptidomimetics (e.g., peptoids), amino acids, amino acid analogs, polynucleotides, polynucleotide analogs, nucleotides, nucleotide analogs, and organic or inorganic compounds (e.g., heteroorganic or organometallic compounds).

Test compounds can be screened individually or in parallel. An example of parallel screening is a high throughput drug screen of large libraries of chemicals. Such libraries of test compounds can be generated or purchased, e.g., from Chembridge Corp., San Diego, CA. Libraries can be designed to cover a diverse range of compounds. For example, a library can include 500, 1000, 10,000, 50,000, or 100,000 or more unique compounds. Alternatively, prior experimentation and anecdotal evidence can suggest a class or category of compounds of enhanced potential. A library can be designed and synthesized to cover such a class of chemicals.

The synthesis of combinatorial libraries is well known in the art and has been reviewed (see, e.g., Gordon et al., J. Med. Chem., 37:1385-1401, (1994); DeWitt, and Czarnik, Acc. Chem. Res., 29:114, (1996); Armstrong, et al., Acc. Chem. Res., 29:123, (1996); Ellman, J. A. Acc. Chem. Res., 29:132, (1996); Gordon, et al., Acc. Chem. Res., 29:144 (1996); Lowe, G. Chem. Soc. Rev., 309 (1995); Blondelle et al. Trends Anal. Chem., 14:83 (1995); Chen, et al., J. Am. Chem. Soc., 116:2661 (1994); U.S. Patents Nos. 5,359,115, 5,362,899, and 5,288,514; and PCT Publication Nos. WO 92/10092, WO 93/09668, WO 91/07087, WO 93/20242, and WO 94/08051).

Libraries of compounds can be screened to determine whether any members of the library have a desired activity and, if so, to identify the active species. Methods of screening combinatorial libraries have been described (see, e.g., Gordon et al., J Med. Chem.*, supra).* After screening, compounds that have a desired activity can be identified by any number of techniques (e.g., mass spectrometry (MS), nuclear magnetic resonance (NMR), matrix-assisted laser desorption ionization/time of flight (MALDI-TOF) analysis, and the like). Exemplary assays useful for screening libraries of test compounds are described herein.

### Uses of HDAC3 Inhibitors

HDAC3 inhibitors (e.g., those identified by the methods described herein) can be used prophylactically or as a treatment for various neurological conditions (e.g., neurological conditions associated with frataxin deficiency). More specifically, HDAC3 inhibitors (e.g., those identified by the methods described herein) can be used to delay or prevent the onset of a neurodegenerative or neuromuscular condition, as well as to treat a mammal, such as a human subject, suffering from a neurological condition (e.g., a neurodegenerative or neuromuscular condition). Non-limiting examples of neurodegenerative conditions include, without limitation, fragile X syndrome, Friedreich's ataxia, Huntington's disease, spinocerebellar ataxias, amyotrophic lateral sclerosis, Kennedy's disease, spinal and bulbar muscular atrophy and Alzheimer's disease. Non-limiting examples of neuromuscular conditions include spinal muscular atrophy and myotonic dystrophy.

Mammals, e.g. humans, to which HDAC3 inhibitors can be administered include those suffering from the conditions discussed above as well as those who are at risk for developing the above conditions. A mammal at risk for developing a neurodegenerative condition can be identified in numerous ways, including, for example, first determining (1) the length, extent, and/or number of repeats of particular nucleic acid sequences (e.g., a frataxin gene sequence, a huntingtin gene sequence, an ataxin gene sequence, a fragile X mental retardation *(FMR1)* gene sequence, a dystrophia myotonica protein kinase *(DMPK)* gene sequence, or an androgen receptor gene sequence) in the individual's genome; the degree of acetylation of core histones; or the expression level of a particular mRNA or protein (e.g., frataxin, huntingtin, brain derived neurotrophic factor *(BDNF),* peroxisome proliferator-activated receptor-gamma, coactivator 1, alpha *(PGC1A),* ataxin, fragile X mental retardation *(FMR1),* dystrophia myotonica protein kinase *(DMPK),* or androgen receptor), and then (2) comparing it with that of a normal individual (see Riley et al., 2006, Genes Dev., 20:2183-92; Tan et al., 2005, Expert Rev. Mol. Diagn., 5:101-109; Everett et al., 2004, Brain, 127:2385-2405; Monckton et al., 1995, Circulation, 91:513-520; and Caskey et al., 1992, Science, 256:784-789).

An individual at risk for developing a neurodegenerative or neuromuscular condition is one who has an aberrant number of repeats of a particular nucleic aid sequence, degree of acetylation of core histones or expression of a particular gene. For example, a mammal at risk for developing Friedreich's ataxia can be identified by determining the length, extent or number of repeats of a GAA triplet in the first intron of the frataxin gene. A mammal can be considered at risk for Friedreich's ataxia if the above analysis indicates that there are more than 34 repeats of the GAA triplet (e.g., more than 40, 50, 60, 66 or 70 repeats of the GAA triplet). A mammal at risk for Friedreich's ataxia can also be identified by determining the levels of frataxin mRNA or protein expressed in the mammal. A mammal would be at risk for Friedreich's ataxia if the levels of frataxin mRNA or protein are lower than the level normally observed in a healthy individual such as for example, an unaffected sibling.

A HDAC3 inhibitor can be administered to an animal or cellular model of a neurological condition. Exemplary models are reviewed in Bates and Gonitel, 2006, Mol. Biotechnol., 32:147-158; Puccio, 2007, Handb. Exp. Pharmacol., 178:365-375; Bates and Hay, 2004, Methods Mol. Biol., 277:3-15; Wansink and Wieringa, 2003, Cytogenet. Genome Res., 100:230-421; Merry et al., 2005, NeuroRx, 2:471-479; Gu and Nelson, 2003, Cytogenet. Genome Res., 100:129-139; Hoogeveen et al., 2002, Microsc. Res. Tech., 57:148-155; Gardian, 2006, Ideggyogy Sz., 59:396-399; Li et al., 2005, NeuroRx, 2:447-464; Levine et al., 2004, Trends Neurosci., 27:691-697; Everett and Wood, 2004, Brain, 127:2385-2405; Outeiro and Muchowski, 2004, J. Mol. Neurosci., 23:49-60; Beal and Ferrante, Nat. Rev. Neurosci., 5:373-384; Link, 2001, Mech. Ageing Dev., 122:1639-49; Heintz and Zoghbi, 2000, Annu. Rev. Physiol., 62:779-802; Martin, 2007, Rev. Neurosci., 18:115-136; Cauchi and van den Heuvel, 2006, Neurodegener. Dis., 3:338-356; Grieb, 2004, Folia Neuropathol., 42:239-248; Robertson et al., 2002, Biochimie, 84:1151-60; Newman et al., 2007, Biochim. Biophys. Acta, 1772:285-297; Van Dam and De Deyn, 2006, Nat. Rev. Drug Discov., 5:956-970; and Shaughnessy et al., J. Mol. Neurosci., 24:23-32.

The amount of HDAC3 inhibitor to be administered to the mammal can be any amount appropriate to restore the level of histone acetylation, or the level of mRNA or protein expression, in the afflicted mammal to that typical of a healthy individual such as an unaffected sibling. The amount of the HDAC3 inhibitor to be administered can be an effective dose or an appropriate fraction thereof, if administration is performed serially. Such amounts will depend on individual patient parameters including age, physical condition, size, weight, the condition being treated, the severity of the condition, and any concurrent treatment. For example, the effective dose range that is necessary to prevent or delay the onset of the neurodegenerative condition can be lower than the effective dose range for inhibiting the progression of the condition being treated. Factors that determine appropriate dosages are well known to those of ordinary skill in the art and can be addressed with routine experimentation. For example, determination of the physicochemical, toxicological, and pharmacokinetic properties can be made using standard chemical and biological assays and through the use of mathematical modeling techniques known in the chemical, pharmacological, and toxicological arts. The therapeutic utility and dosing regimen can be extrapolated from the results of such techniques and through the use of appropriate pharmacokinetic and/or pharmacodynamic models. The precise amount of HDAC3 inhibitor administered to a patient will be the responsibility of the attendant physician.

In particular, HDAC3 inhibitors (e.g., those identified by the methods described herein) can be administered orally or by injection at a dose of from 0.1 to 30 mg per kg weight of the mammal, typically 2 to 15 mg/kg weight of the mammal. The dose range for adult humans is generally from 8 to 2,400 mg/day, e.g., from 35 to 1,050 mg/day. If the salt of the compound is administered, then the amount of salt administered is calculated in terms of the base.

HDAC3 inhibitors (e.g., those identified by the methods described herein) can be administered in numerous ways. For example, the HDAC3 inhibitors can be administered orally, rectally, topically, or by intramuscular, intraperitoneal subcutaneous or intravenous injection. Preferably, the inhibitors are administered orally or by injection. Other routes include intrathecal administration directly into spinal fluid and direct introduction onto, in the vicinity of, or within the target cells. The route of administration will depend on the condition being treated and its severity.

Toxicity and therapeutic efficacy of HDAC3 inhibitors (e.g., identified by the methods described herein) can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit high therapeutic indices are preferred. In another embodiment, the therapeutic index can be estimated by assaying the HDAC3 specific inhibitory activity of a HDAC3 inhibitor (the HDAC3 IC₅₀) as compared to the growth inhibitory activity of the HDAC3 inhibitor on a cell in vitro, e.g., a HepG2 cell or other cell line (the growth IC₅₀). The ratio between the growth inhibitory (e.g., cytotoxic or cytostatic) effect and the HDAC3 specific inhibitory effect provides an estimate of the therapeutic index.

### Pharmaceutical Compositions

HDAC3 inhibitors identified by the methods described herein can be administered neat or formulated as pharmaceutical compositions. Pharmaceutical compositions include an appropriate amount of the HDAC inhibitor in combination with an appropriate carrier as well as other useful ingredients.

Pharmaceutical compositions of HDAC3 inhibitors suitable for oral administration can be in the form of (1) discrete units such as capsules, cachets, tablets, or lozenges each containing a predetermined amount of the HDAC3 inhibitor; (2) a powder or granules; (3) a bolus, electuary, or paste; (4) a solution or a suspension in an aqueous liquid or a non-aqueous liquid; or (5) an oil-in-water liquid emulsion or a water-in-oil liquid emulsion. Compositions suitable for topical administration in the mouth, for example buccally or sublingually, include lozenges. Compositions suitable for parenteral administration include aqueous and non-aqueous sterile suspensions or injection solutions. Compositions suitable for rectal administration can be presented as a suppository.

Pharmaceutical compositions of HDAC3 inhibitors can be formulated using a solid or liquid carrier. The solid or liquid carrier should be compatible with the other ingredients of the formulation and not deleterious to the recipient. If the pharmaceutical composition is in tablet form, then the HDAC3 inhibitor is mixed with a carrier having the necessary compression properties in suitable proportions and compacted in the shape and size desired. If the composition is in powder form, the carrier is a finely divided solid in admixture with the finely divided active ingredient. The powders and tablets can contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins. A solid carrier can include one or more substances that can act as flavoring agents, lubricants, solubilizers, suspending agents, fillers, glidants, compression aids, binders or tablet-disintegrating agents. A suitable carrier can also be an encapsulating material.

If the composition is a solution, suspension, emulsion, syrup, elixir, or pressurized composition, then liquid carriers can be used. In this case, the HDAC3 inhibitor is dissolved or suspended in a pharmaceutically acceptable liquid carrier. Suitable examples of liquid carriers for oral and parenteral administration include (1) water; (2) alcohols, e.g. monohydric alcohols and polyhydric alcohols such as glycols, and their derivatives; and (3) oils, e.g. fractionated coconut oil and arachis oil. For parenteral administration, the carrier can also be an oily ester such as ethyl oleate and isopropyl myristate. Liquid carriers for pressurized compositions include halogenated hydrocarbon or other pharmaceutically acceptable propellant. The liquid carrier can contain other suitable pharmaceutical additives such as solubilizers; emulsifiers; buffers; preservatives; sweeteners; flavoring agents; suspending agents; thickening agents; colors; viscosity regulators; stabilizers; osmo-regulators; cellulose derivatives such as sodium carboxymethyl cellulose; antioxidants; and bacteriostatics. Other carriers include those used for formulating lozenges such as sucrose, acacia, tragacanth, gelatin and glycerin as well as those used in formulating suppositories such as cocoa butter or polyethylene glycol.

If the composition is to be administered intravenously or intraperitoneally by infusion or injection, solutions of the HDAC3 inhibitor can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of microorganisms. The composition suitable for injection or infusion can include sterile aqueous solutions or dispersions or sterile powders comprising the active ingredient, which are adapted for the extemporaneous preparation of sterile injectable or infusible solutions or dispersions, optionally encapsulated in liposomes. In all cases, the ultimate dosage form should be sterile, fluid and stable under the conditions of manufacture and storage. The liquid carrier or vehicle can be a solvent or liquid dispersion medium as described above. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin. Sterile injectable solutions are prepared by incorporating the HDAC3 inhibitor in the required amount in the appropriate solvent with various of the other ingredients enumerated above, as required, followed by filter sterilization. In the case of sterile powders for the preparation of sterile injectable solutions, the preferred methods of preparation are vacuum drying and the freeze-drying techniques, which yield a powder of the HDAC3 inhibitor, plus any additional desired ingredient present in the previously sterile-filtered solutions.

Pharmaceutical compositions can be in unit-dose or multi-dose form or in a form that allows for slow or controlled release of the HDAC3 inhibitor. Each unit-dose can be in the form of a tablet, capsule or packaged composition such as, for example, a packeted powder, vial, ampoule, prefilled syringe or sachet containing liquids. The unit-dose form also can be the appropriate number of any such compositions in package form. Pharmaceutical compositions in multi-dose form can be in packaged in containers such as sealed ampoules and vials. In this case, the HDAC3 inhibitor can be stored in a freeze-dried (lyophilized) condition requiring only the addition of a sterile liquid carrier immediately prior to use. In addition, extemporaneous injection solutions and suspensions can be prepared from sterile powders, granules and tablets of the kind previously described.

### EXAMPLES

### Example 1. RGFA8 is a Specific Inhibitor of HDAC3

To determine whether RGFA8 (*N*¹-(2-aminophenyl)-*N*⁷-*p*-tolylheptanediamide; WO 2007/058927) was specific for any particular HDAC or subset of HDACs, the activities of RGFA8 and known HDAC inhibitor trichostatin A (TSA) were tested on a panel of individual purified HDAC enzymes and a nuclear extract, which contained a mixture of HDACs. HDAC enzyme inhibition assays were performed using purified HDACs 1-10 essentially as described in Beckers et al., 2007, Int. J. Cancer., 121:1138-48 and Pérez-Balado et al., 2007, J. Med. Chem., 50:2497-2505. Inhibition assays using nuclear extract were performed essentially as described in Herman et al., 2006, Nat. Chem. Biol., 2:551-558. Briefly, the purified HDACs or nuclear extract were incubated with an acetylated substrate in the absence of the compound to be assayed and with increasing concentrations of the compound. The rate of substrate deacetylation was measured under each condition, and half-maximal inhibitory concentration with regard to each HDAC was determined by standard means.

RGFA8 was most active on HDAC3, with a half-maximal inhibitory concentration (IC₅₀) of 0.20 ± 0.23 µM (Table 1). At least 10-fold lesser activity was observed by RGFA8 on other HDACs or on nuclear extract. Although TSA was found to be a more potent inhibitor of HDAC3 than RGF8, TSA had greater inhibitory activity on HDAC6 (IC₅₀ of 0.0014 ± 0.0006) and HDAC1 (IC₅₀ of 0.0067 ± 0.0011) as compared to HDAC3 (IC₅₀ of 0.0096 ± 0.0071). Sub-micromolar inhibition by TSA was observed for all HDACs tested.

**Table 1. Inhibition of HDAC Activity by RGFA8 and TSA**

| **Enzyme or Extract** | **IC₅₀ (µM)^{a}** | |
|---|---|---|
| | **RGFA8** | **TSA** |
| HDAC1 | 3.05 | 0.0067 |
| HDAC2 | 3.73 | 0.0148 |
| **HDAC3** | 0.74 | 0.0096 |
| HDAC4 | 93.8 | 0.0348 |
| HDAC5 | 7.94 | 0.0125 |
| HDAC6 | > 80.1 | 0.0014 |
| HDAC7 | > 100 | 0.197 |
| HDAC8 | > 100 | 0.165 |
| HDAC9 | > 100 | 0.0701 |
| HDAC10 | > 66.2 | 0.0228 |
| Nuclear Extract | 6.00 | 0.0012 |

This example demonstrates that RGFA8 specifically inhibits HDAC3 as compared to other human HDACs. HDAC inhibitors that are specific for HDAC3 can be used to treat neurological conditions (e.g., Friedreich's ataxia).

### Example 2. RGFA8 Increases Expression of Frataxin

To determine whether RGFA8 or other compounds could increase expression of frataxin, human lymphocytes isolated from peripheral blood from normal donors were incubated with 1-30 µM RGFA8. Frataxin mRNA levels were measured with quantitative RT-PCR and normalized to expression levels of the housekeeping gene GADPH (Herman et al., Nat. Chem. Biol., 2:551-558, 2006).

RGFA8 increased expression of frataxin in normal lymphocytes or patient lymphocytes at all concentrations tested, with a maximum observed increase of about 16-fold compared to vehicle control (FIG. 1, normal lymphocytes). This example indicates that RGFA8 could be used to treat patients with Friedreich's ataxia by increasing frataxin expression.

### Example 3. Screen for HDAC3 Inhibitors

A chemical library was screened to identify compounds that specifically inhibited HDAC3, relative to other HDACs. Briefly, a chemical library of test compounds was created by standard organic chemistry methods, and the inhibitory activity of the compounds on purified HDACs 1-10 was determined (see Example 1). More than 20 compounds were identified. Exemplary compounds, their inhibitory activities for HDAC1, HDAC2, HDAC3, HDAC5, and their growth inhibitory activity on HepG2 cells are presented in Table 2. HDAC inhibitory activities were measured essentially as described in Example 1. Growth inhibition of HepG2 cells was measured by adding serial dilutions of the compounds to HepG2 cells at a density of 5×10⁴ cells/ml, and incubating the mixture for 72 hours at 37 °C, 5% CO₂. The viable cells were then measured using a CellTiter 96™ AQᵤₑₒᵤₛ One Solution cell proliferation assay (Promega, Madison, WI). The activities of RGFA8 and the known HDAC inhibitor MS-275 are also presented.

**Table 2. Activity of Identified HDAC3 Inhibitors**

| **Compound** | **Structure** | **IC₅₀ (µM)** | | | | **HepG2 Growth IC₅₀ (µM)** |
|---|---|---|---|---|---|---|
| | | **HDAC1** | **HDAC2** | **HDAC3** | **HDAC5** | |
| MS-275 | 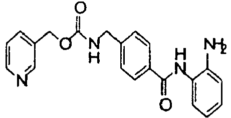 | 3.2 | 0.72 | 0.59 | 5.5 | 4.00 |
| RGFA8 | 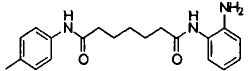 | 3.05 | 3.73 | 0.74 | 7.94 | 10.00 |
| 21 | 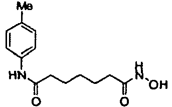 | 1.19 | 2.92 | 0.32 | 1.63 | 10.00 |
| 6 | 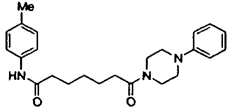 | 4.2 | 5.9 | 9.1 | 18.2 | 112 |

Relative inhibitory activities for the compounds were determined by dividing the IC₅₀ for HDACs 1, 2, and 5 by the IC₅₀ for HDAC3 (Table 3). The estimated therapeutic index for each compound was determined by dividing the HepG2 growth IC₅₀ by the IC₅₀ for HDAC3 activity (Table 3). Further, the compounds were assayed by quantitative RT-PCR for its activity to increase expression of frataxin (FXN1) mRNA in human lymphocytes isolated from peripheral blood of normal donors (see Example 2). Briefly, the compounds were added to lymphocytes at a concentration of 10 µM, and increase in expression of *FXN1* mRNA was determined compared to vehicle control. The majority of the identified compounds increased frataxin mRNA expression at a concentration of 10 µM (Table 3), indicating that these compounds can be useful in treatment of Friedrich's ataxia and other neurological disorders described herein. Compound 6, however, did not show strong inhibition of HDAC3 and was not active to increase frataxin expression. MS-275 did appreciably increase frataxin mRNA expression, but may be too cytotoxic for long-term use. Compound 21 exemplifies a compound with strong HDAC3 inhibitory activity and relatively low cytotoxicity that is effective to increase frataxin expression.

**Table 3. Relative HDAC Inhibition Activities and Effect on FXN1 Expression**

| **Compound** | **Relative Inhibitory Activity** | | | | **Fold Increase *FXN1* Expr. (10 µM)** |
|---|---|---|---|---|---|
| | **HDAC 1/3** | **HDAC 2/3** | **HDAC 5/3** | **HepG2/ HDAC3** | |
| MS-275 | 5.3 | 1.2 | 9.3 | 6.7 | 8.0 |
| RGFA8 | 4.1 | 5.0 | 10.7 | 13 | 2.7 |
| 21 | 3.7 | 9.1 | 5.1 | 31 | 2.0 |
| 6 | 0.45 | 0.65 | 2.0 | 12.3 | none |

## Claims

1. A method of identifying a candidate compound for treatment of a neurological condition associated with a frataxin deficiency, the method comprising:
assaying a first activity of a test compound to inhibit histone deacetylase activity of a histone deacetylase 3 (HDAC3);
assaying a second activity of the test compound to inhibit histone deacetylase activity of a class I or class II histone deacetylase other than the HDAC3; and
identifying the test compound as a candidate compound for treatment of a neurological condition associated with a frataxin deficiency if the first activity of the test compound is greater than the second activity of the test compound.

2. The method of claim 1, wherein the second activity is the activity of the test compound to inhibit histone deacetylase activity of a class I histone deacetylase other than the HDAC3.

3. The method of claim 1, wherein the method comprises:
assaying a first activity of a test compound to inhibit histone deacetylase activity of a histone deacetylase 3 (HD AC3);
assaying a second activity of the test compound to inhibit histone deacetylase activity of a histone deacetylase 1 (HDAC1);
assaying a third activity of the test compound to inhibit histone deacetylase activity of a histone deacetylase 2 (HDAC2);
assaying a fourth activity of the test compound to inhibit histone deacetylase activity of a histone deacetylase 8 (HDAC8); and
identifying the test compound as a candidate compound for treatment of a neurological condition if the first activity of the test compound is greater than each of the second, third, and fourth activities of the test compound.

4. The method of claim 2, wherein the class I histone deacetylase other than a HDAC3 is a histone deacetylase 1 (HDAC1), a histone deacetylase 2 (HDAC2), or a histone deacetylase 8 (HDAC8).

5. The method of claim 1 or 2, wherein the HDAC3 is a human HDAC3 or wherein the histone deacetylase other than a HDAC3 is a human histone deacetylase.

6. The method of claim 3, wherein each of the HDAC3, HDAC1, HDAC2, and HDAC8 are human.

7. The method of claim 1 to 3, wherein the first activity is at least about 2-fold greater than the second activity or than each of the second, third, and fourth activities.

8. The method of claim 1 to 3, wherein the neurological condition is associated with expansion of a triplet repeat.

9. The method of claim 8, wherein the neurological condition is Friedreich's ataxia.

10. The method of claim 1, wherein the method comprises:
assaying a activity of a test compound to inhibit histone deacetylase activity of a histone deacetylase 3 (HDAC3);
assaying a set of activities of the test compound to inhibit histone deacetylase activity of each of histone deacetylases 1, 2, 4, 5, 6, 7, 8, 9, and 10; and
identifying the test compound as a candidate compound for treatment of a neurological condition if the first activity of the test compound is greater than each activity of the set of activities of the test compound.

11. The method of claim 1, 2, 7, or 10, further comprising assaying the activity of the candidate compound to increase expression of a gene whose expression is decreased in the neurological condition, in particular frataxin.

12. The method of claim 1 or 2, wherein the first activity is at least about 2-fold the activity greater than the second activity.

## Patentansprüche

1. Verfahren zum Identifizieren einer Kandidatenverbindung für die Behandlung einer mit Frataxindefizienz assoziierten neurologischen Erkrankung, wobei das Verfahren Folgendes umfasst:
Testen einer ersten Hemmaktivität einer Prüfverbindung auf die Histondesacetylaseaktivität einer Histondesacetylase 3 (HDAC3);
Testen einer zweiten Hemmaktivität der Prüfverbindung auf die Histondesacetylaseaktivität einer Klasse-I- oder Klasse-II-Histondesacetylase, bei der es sich nicht um HDAC3 handelt; und
Identifizieren der Prüfverbindung als Kandidatenverbindung für die Behandlung einer mit Frataxindefizienz einhergehenden neurologischen Erkrankung, wenn die erste Aktivität der Testverbindung stärker als die zweite Aktivität der Testverbindung ist.

2. Verfahren nach Anspruch 1, wobei es sich bei der zweiten Aktivität um die Hemmaktivität der Prüfverbindung auf die Histondesacetylaseaktivität einer Klasse-I-Histondesacetylase, bei der es sich nicht um die HDAC3 handelt, handelt.

3. Verfahren nach Anspruch 1, wobei das Verfahren Folgendes umfasst:
Testen einer ersten Hemmaktivität einer Prüfverbindung auf die Histondesacetylaseaktivität einer Histondesacetylase 3 (HDAC3);
Testen einer zweiten Hemmaktivität einer Prüfverbindung auf die Histondesacetylaseaktivität einer Histondesacetylase 1 (HDAC1);
Testen einer dritten Hemmaktivität einer Prüfverbindung auf die Histondesacetylaseaktivität einer Histondesacetylase 2 (HDAC2);
Testen einer vierten Hemmaktivität einer Prüfverbindung auf die Histondesacetylaseaktivität einer Histondesacetylase 8 (HDAC8); und
Identifizieren der Prüfverbindung als Kandidatenverbindung für die Behandlung einer neurologischen Erkrankung, wenn die erste Aktivität der Prüfverbindung stärker als jede der zweiten, dritten und vierten Aktivitäten der Prüfverbindung ist.

4. Verfahren nach Anspruch 2, wobei es sich bei der Klasse-I-Histondesacetylase, bei der es sich nicht um eine HDAC3 handelt, um eine Histondesacetylase 1 (HDAC1), eine Histondesacetylase 2 (HDAC2) oder eine Histondesacetylase 8 (HDAC8) handelt.

5. Verfahren nach Anspruch 1 oder 2, wobei es sich bei der HDAC3 um eine Human-HDAC3 handelt oder wobei es sich bei der Histondesacetylase, bei der es sich nicht um eine HDAC3 handelt, um eine Human-HistonDesacetylase handelt.

6. Verfahren nach Anspruch 3, wobei jede aus der Reihe HDAC3, HDAC1, HDAC2 und HDAC8 human ist.

7. Verfahren nach Anspruch 1 bis 3, wobei die erste Aktivität mindestens um das ungefähr 2-fache größer als die zweite Aktivität oder als jede der zweiten, dritten und vierten Aktivität ist.

8. Verfahren nach Anspruch 1 bis 3, wobei die neurologische Erkrankung mit einer Triplett-Repeat-Expansion einhergeht.

9. Verfahren nach Anspruch 8, wobei es sich bei der neurologischen Erkrankung um Friedreichs Ataxie handelt.

10. Verfahren nach Anspruch 1, wobei das Verfahren Folgendes umfasst:
Testen einer Hemmaktivität einer Prüfverbindung auf die Histondesacetylaseaktivität einer Histondesacetylase 3 (HDAC3);
Testen eines Satzes von Hemmaktivitäten der Prüfverbindung auf die Histondesacetylaseaktivität von jeder der Histondesacetylasen 1, 2, 4, 5, 6, 7, 8, 9 und 10; und
Identifizieren der Testverbindung als Kandidatenverbindung für die Behandlung einer neurologischen Erkrankung, wenn die erste Aktivität der Prüfverbindung stärker als jede Aktivität des Satzes der Aktivitäten der Prüfverbindung ist.

11. Verfahren nach Anspruch 1, 2, 7 oder 10, das weiterhin Folgendes umfasst: Testen der expressionserhöhenden Aktivität der Kandidatenverbindung auf ein Gen, dessen Expression bei der neurologischen Erkrankung verringert ist, insbesondere Frataxin.

12. Verfahren nach Anspruch 1 oder 2, wobei die erste Aktivität mindestens um das 2-fache stärker ist als die zweite Aktivität.

## Revendications

1. Procédé d'identification d'un composé candidat pour le traitement d'une affection neurologique associée à une déficience en frataxine, le procédé comprenant:
le dosage d'une première activité d'un composé d'essai pour inhiber l'activité histone désacétylase d'une histone désacétylase 3 (HDAC3) ;
le dosage d'une deuxième activité du composé d'essai pour inhiber l'activité histone désacétylase d'une histone désacétylase de classe I ou de classe II autre que HDAC3 ; et
l'identification du composé d'essai en tant que composé candidat pour le traitement d'une affection neurologique associée à une déficience en frataxine si la première activité du composé d'essai est supérieure à la deuxième activité du composé d'essai.

2. Procédé de la revendication 1, dans lequel la deuxième activité est l'activité du composé d'essai pour inhiber l'activité histone désacétylase d'une histone désacétylase de classe I autre que HDAC3.

3. Procédé de la revendication 1, dans lequel le procédé comprend:
le dosage d'une première activité d'un composé d'essai pour inhiber l'activité histone désacétylase d'une histone désacétylase 3 (HDAC3) ;
le dosage d'une deuxième activité du composé d'essai pour inhiber l'activité histone désacétylase d'une histone désacétylase 1 (HDAC1);
le dosage d'une troisième activité du composé d'essai pour inhiber l'activité histone désacétylase d'une histone désacétylase 2 (HDAC2) ;
le dosage d'une quatrième activité du composé d'essai pour inhiber l'activité histone désacétylase d'une histone désacétylase 8 (HDAC8) ; et
l'identification du composé d'essai en tant que composé candidat pour le traitement d'une affection neurologique si la première activité du composé d'essai est supérieure à chacune des deuxième, troisième et quatrième activités du composé d'essai.

4. Procédé de la revendication 2, dans lequel l'histone désacétylase de classe I autre qu'un HDAC3 est une histone désacétylase 1 (HDAC1), une histone désacétylase 2 (HDAC2), ou une histone désacétylase 8 (HDAC8).

5. Procédé de la revendication 1 ou 2, dans lequel le HDAC3 est un HDAC3 humain ou dans lequel l'histone désacétylase autre que HDAC3 est une histone désacétylase humaine.

6. Procédé de la revendication 3, dans lequel chacun des HDAC3, HDAC1, HDAC2, et HDAC8 sont un humain.

7. Procédé de la revendication 1 à 3, dans lequel la première activité est au moins environ 2 fois plus élevée que la deuxième activité ou que chacune des deuxième, troisième et quatrième activités.

8. Procédé de la revendication 1 à 3, dans lequel l'affection neurologique est associée à l'expansion d'une répétition de triplets.

9. Procédé de la revendication 8, dans lequel l'affection neurologique est l'ataxie de Friedreich.

10. Procédé de la revendication 1, dans lequel le procédé comprend:
le dosage d'une activité d'un composé d'essai pour inhiber l'activité histone désacétylase d'une histone désacétylase 3 (HDAC3) ;
le dosage d'un ensemble d'activités du composé d'essai pour inhiber l'activité histone désacétylase de chacune des histones désacétylases 1, 2, 4, 5, 6, 7, 8, 9 et 10 ; et
l'identification du composé d'essai en tant que composé candidat pour le traitement d'une affection neurologique si la première activité du composé d'essai est supérieure à chaque activité de l'ensemble d'activités du composé d'essai.

11. Procédé de la revendication 1, 2, 7 ou 10, comprenant en outre le dosage de l'activité du composé candidat pour augmenter l'expression d'un gène dont l'expression est diminuée dans l'affection neurologique, en particulier la frataxine.

12. Procédé de la revendication 1 ou 2, dans lequel la première activité est au moins environ 2 fois supérieure à la deuxième activité.
